# EUROPEAN PATENT SPECIFICATION

(11) **EP 1 703 804 B1**
(45) Date of publication and mention of the grant of the patent: **30.01.2008**
(21) Application number: 04803022.5
(22) Date of filing: 16.12.2004
(51) Int. Cl.: A23J 3/00, A23J 3/34

(54) **A PLANT FOR ENZYMATIC HYDROLYSIS OF A BATCH OF ANIMAL OR VEGETABLE COMPONENTS AND A METHOD FOR USE OF THE PLANT**
ANLAGE FÜR DIE ENZYMATISCHE HYDROLYSE VON EINER CHARGE TIER- ODER PFLANZENBESTANDTEILE, SOWIE VERFAHREN ZUR VERWENDUNG DER ANLAGE
INSTALLATION POUR L'HYDROLYSE ENZYMATIQUE D'UN LOT DE COMPOSANTS ANIMAUX OU VEGETAUX ET PROCEDE D'UTILISATION DE L'INSTALLATION

(30) Priority: 18.12.2003 DK 200301879
(43) Date of publication of application: 27.09.2006
(73) Proprietor: Danflavour ApS, 2800 Lyngby (DK)
(72) Inventor: S RENSEN, Stig Voldbjerg, DK-2800 Lyngby (DK)
(74) Representative: Holme Patent A/S
(86) International application number: PCT/DK2004/000869
(87) International publication number: WO 2005/058059

(56) References cited:
- FR-A1- 2 352 498
- US-A- 4 378 311
- US-A- 5 162 129

## Description

The invention relates to a plant for enzymatic hydrolysis of a batch of at least partly hydrolysable animal or vegetable components or a mixture of these components, and of the kind comprising a reactor with a mixer and at least one outlet, where the reactor has an open top section and a bottom.

A large part of the food industry, such as slaughterhouses and the finished product industry, produce large quantities of waste products through the processing of for example meat, fish, and poultry. Such waste products are removed by burning or they can, to a certain extent, be transformed and be used again to for example bone meal or animal food.

The hydrolysable part of the waste products can however be hydrolysed and used in the production of for example aromatics, stocks, and soups.

A continual hydrolysis demands large space-demanding plants, large investments and large quantities of raw material in order to be profitable. With smaller or small raw material quantities smaller batch plants advantageously can be used, as described for example in FR 2 352 498.

The known small batch plants consist of a vertical reactor with a heating jacket and a mixer which keeps the reaction mixture mixed. The reactor typically has a conical bottom with a bottom outlet, through which the finished hydrolysed mass, that is to say the mixture of hydrolysate, fat, and bone, is removed in one step for subsequent separation in for example a decanter. One of the disadvantages of these known plants is that the bottom outlet is easily clogged since the bones are packed together in the bottom outlet, while the reactor is emptied. In some batch reactors, it is attempted to solve this problem by means of an auger rotating over the bottom outlet. The auger lifts, to a certain amount, the bones away from the bottom outlet but only partly lowers the risk of clogging of the bottom outlet since the auger rotates relatively slowly.

As a consequence of the operating problems of the batch plant, the hydrolysable waste products are only utilized in a limited amount.

The waste products from meat processing are a directly available raw material, which furthermore also is expensive to get rid of. Utilisation of this waste for production of a hydrolysate, which can be used as for example soups, stock, and aroma-concentrates, can therefore be an advantageous source of income, as long as the operation of the batch plant can be optimised.

In a first aspect according to the present invention a batch plant is provided for enzymatic production of a hydrolysate, where the batch plant has a simple, reliable operation, and is easier to maintain and use than previously known.

In a second aspect according to the present invention a batch plant is provided for utilisation of enzymatic hydrolysable byproducts from foodstuffs and foodstuff processing.

The novel and unique solution whereby this is achieved according to the present invention consists in that the reactor is formed as a round bottom trough with two opposite side walls, a first and a second opposite end walls, a rotatable axle suspended between the end walls at a distance from the bottom and at least one mixer arranged on the axle.

Sedimentation and clumping of the solid phase from a reaction mixture, which for example consists of water, meat containing components, and enzymes, will in these conventional plants mean that there easily forms local pockets in the batch of untreated raw material which significantly reduced the output and can give bacteriological problems. Furthermore the bottom outlet can be easily blocked when emptying the reactor.

This significant operational problem is advantageously eliminated in the batch plant according to the present invention by forming the reactor as a round bottom trough and carefully dimensioning the trough, the outlets placement, the axle and the at least one mixer in relation to each other. A longitudinal reactor formed in this way can advantageously give room to a number of mixers spaced along the axle. In this way a better mixing is ensured and thereby faster hydrolysis. The round bottom ensures that the solid components have a larger area to rest on than in the known reactors, and the risk for the liquid pressure to force components into the outlet are minimized.

An suitable reaction temperature and temperature control can advantageously be maintained, if the reaction chamber for example is connected to a heating jacket. The reaction chamber can alternatively be heated up with a submerged heating element, e.g. imbedded in the axle. The walls of the trough can also be electrically heated, or the mixers can function as heating elements. Such alternative heating forms are comprised within the scope of the invention. In order to further ensure that the supplied heat is not lost to the environment, and thereby decrease the reaction speed of the hydrolysis, with thereby following decreased output, it is expedient to insulate the reaction chamber.

It is very important that the mixer can reach or pull along the reaction mixture on the bottom of the trough such that the reaction mixture in the entire reaction time is whirled up in a fluid bed with optimal continuous heat distribution and contact with enzyme as long as the hydrolysis process shall take place.

This is ensured, according to the invention, in that the mixer extends mainly symmetrically around the axis of the axle towards the bottom of the trough with a length which is equal to or a little smaller than the distance between the axis of the axle and the deepest point of the trough.

During the aqueous enzymatic hydrolysis process of a batch of hydrolysable animal or vegetable components, such as for example bone waste from the de-boning of chicken or fish, aqueous phase is formed an and a fat phase, which floats on the aqueous phase. Since it is desired to maintain this phase separation and to ensure that the mixers do not pull the fat particles from the fat phase down into the aqueous phase, the depth of the trough must be so much larger than the total length of mixer that the fat phase overlay the rotation top point of the at least one mixer with a distance which is larger than zero, preferably 5% larger and most preferably 10% larger.

It is an important aspect of the batch plant according to the present invention, that the trough is so deep that it is possible, at any time, to fill it so much that the produced fat phase overlies the rotation top point of the mixers so much, that this fat phase is not affected by the currents and the turbulences in the reactor during the rotation of the mixers.

The rotation can either have a constant rotation direction or can have reversing rotation direction in time-determined intervals.

The preferred size of the trough and mixers can be correlated by experiments to the fat content of the waste type, and tabulated such that acquisition of a batch plant according to the present invention is made especially easy, as long as the amount of raw material and its main composition is known. In situations of doubt, the depth of the trough can be chosen with a margin of safety between the expected amount of fat phase and rotation top point of the mixers.

In a preferred, especially effective working embodiment for the batch plant according to the present invention, a number of mixers can be arranged angularly displaced around the axis of the axle on the axle with a mainly equidistant spacing between each other. In this way it is avoided that the reaction mixture sediments during the hydrolysis process.

When the hydrolysis process is finished, the mixers are stopped and the reaction mixture, which consists of an aqueous hydrolysate and particulate material, separates itself naturally as a consequence of sedimentation, into a aqueous hydrolysate phase and a solid phase, which consists of lightly packed particulate material and cleaned bone fragments. The solid phase sediments on the bottom of the reactor and will act as a rough pored filter, which holds the particulate material back which is possibly suspended in the aqueous hydrolysate, reaches this as the first phase is drained off from the reactor through the hydrolysate outlet, which is formed in at least one of the side walls close to the bottom of the trough. As a consequence of the filtering properties of the solid phase, the hydrolysate phase appears transparent without visible suspended material.

The fluid pressure during the draining off will not, as in the known batch plants, press the solid phase towards the outlet during the draining off. The outlet therefore does not get blocked. In the known reactors, where the outlet is arranged in the conical bottom, the solid components are guided by the conical wall directly down into the outlet during the draining off. This can not occur in a round bottom trough. In addition, it is necessary to centrifuge the hydrolysate in the known plants in order to get a clear liquid. This treatment step is also avoided according to the present invention.

As the hydrolysate phase is drained off, the liquid level falls in the reactor and the fat phase nears the solid phase of particulate material and bone fragments.

The main part of the floating, warm fat phase can pass out through the hydrolysate outlet. A part of the fat phase will advantageously rest on or fat encapsulate the particles of the aqueous hydrolysate retained in the solid phase filter, whereby also the drained off fat fraction is without significant content of turbidity.

The draining off of the hydrolysate proceeds advantageously via a hydrolysate outlet which has a minimum diameter of approximately 5 cm or larger, and which is equipped with a first valve.

In an especially expedient embodiment for the present invention, the distance between the end walls of the reactor and the first valve is less than or equal to 50cm, preferably less than or equal to 30cm, and most preferably less than or equal to 15cm. The inventor's own experiments show that in this embodiment a continuous draining off can take place without blocking the outlets.

The solid phase can if desired, be drained off through the first valve, when the belonging outlet pipe has a large enough diameter.

In an additional embodiment for the present invention, the bottom of the trough or the sidewalls can be formed with a second valve or a damper for emptying the solid phase with the particulate material.

By in constant rotation direction rotating the mixers, the shovel blades will push the solid phase towards a second outlet with a second valve, which can for example be a slide valve, in order to thereafter empty the solid phase through the second outlet, when the second valve is opened. If the reactor following this still has a remainder of solid phase, the reactor can be filled up with water and leached by e.g. mixing the washing mixture by rotating the axle in e.g. reversing movements and emptying the wash water and bones through one or both valves. The washed bones can e.g. be reused as calcium phosphate after drying.

The invention also relates to a method for use of the batch plant according to the present invention and both the method and the batch plant will be described in more detail in the following with reference to the accompanying drawings and example, in which
Fig. 1 shows, a perspective view of an empty batch plant according to the present invention,
Fig. 2 shows a cross sectional view according to the line II-II in fig. 1, near the end wall with the hydrolysate outlet, of a charged batch plant in a starting hydrolysis step, and
Fig. 3 shows the same in a final step, where the phase separation has taken place.

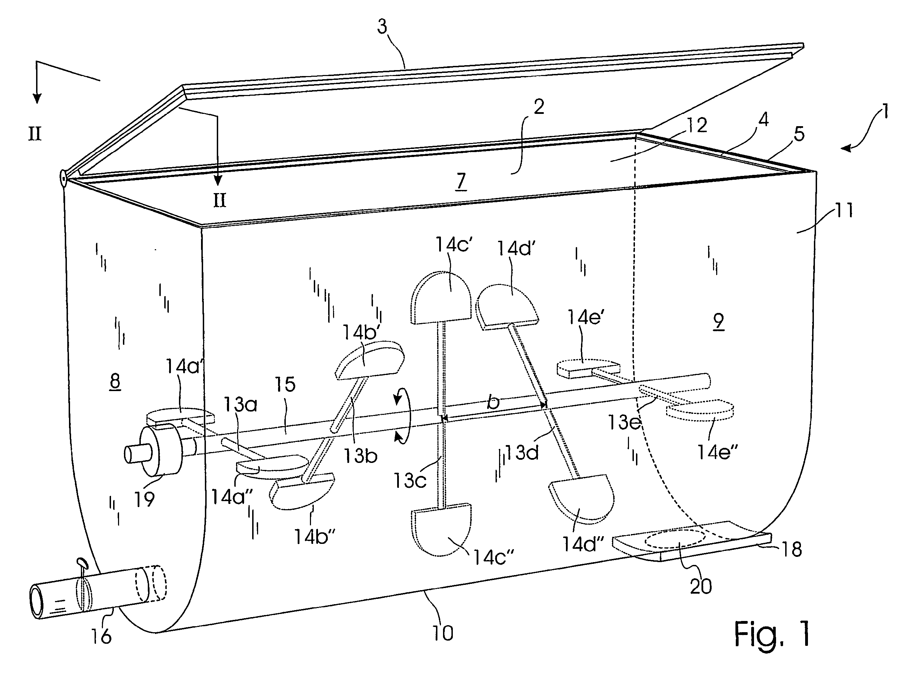

In the following it is assumed that the batch plant 1 shown in fig. 1 is used for proteolytic hydrolysis of a mixture of water and broken up meat bones, in order to produce a concentrated meat soup.

The batch plant 1 consists of a reactor 2 in the form of a trough 2 with a lid 3. The reactor, which typically will be a tank of acid resistant stainless steel , is encapsulated in a heating jacket 4, about which a heat insulating jacket 5 is arranged. The lid 3 is here shown to be a pivoting lid 3, which with a hinge 6 can be brought to cover the open top section 7 of the trough 2. The lid can be insulated in the same way as the trough 2. The reactor 2 has a first end wall 8 and a second end wall 9, a bottom 10 and to oppositely placed side walls 11,12. Between the end walls 8,9 is suspended a rotating axle 15 on which there in the case shown are mounted in total five identical mixers 13a,13b,13c,13d,13e. Each mixer 13 has two terminal shovels 14a',14a",14b',14b",14c',14c",14d',14d", 14e',14e" to keep the reaction mixture free of the bottom 10 of the trough 2. The mixers 13 are in the case shown mounted perpendicularly to the axis of the axle 15 at a mutual distance b and angularly displaced with respect to each other.

In the first end wall 8 is formed a hydrolysate outlet 16 with a valve 17, through which amongst others, aqueous hydrolysate from the reactor 2 is drained off. In the bottom 10 at the opposite second end wall 9 is arranged a slide valve 18 with a bone outlet 20 for emptying off the solid components, such as e.g. cleaned meat bones.

The rotation of the axle 12, and thereby the rotation of the mixers 13, is driven by a motor 19, and the mixing proceeds alternately, in the one and the other rotation direction. Mixing can though within the scope of the invention also occur in the same rotation direction.

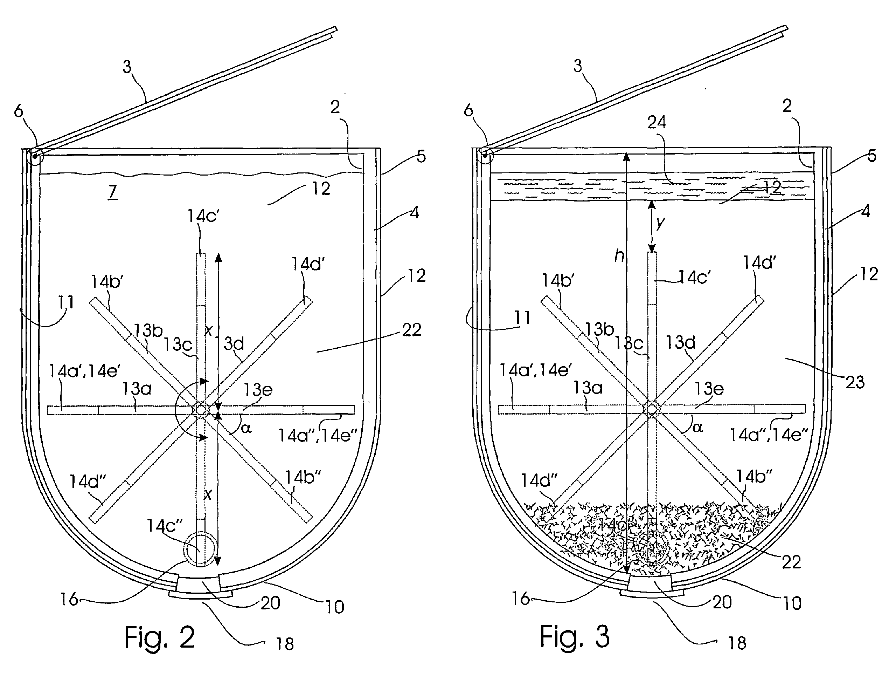

As it is best seen in fig. 2, the shovels 1 of the mixers 13 will alternately pass along the bottom 10 of the trough 2, and the shovels 14 will hereby keep a reaction mixture 21, consisting of e.g. water, chopped up meat bones and enzymes, in constant motion, such that the particulate material is not, or only in a very limited amount allowed to settle.

After a predetermined hydrolysis time at a constant hydrolysis temperature, e.g., at the temperature optimum of the enzyme, the temperature is increased in the heating jacket to a temperature which inactivates the enzyme. The rotation of the mixers is stopped, and the hydrolysed reaction mixture is left, as shown in fig. 3, in a time interval, in which the solid phase 22, which mainly consists of cleaned bones, settles on the bottom of the reactor. The aqueous phase 23, with the achieved content of proteins, peptides and amino acids, settles on and above the solid phase and it its pore volume, and the fat fase 24 settles on the surface of the aqueous phase 23 in a distance y to a rotation top point of a mixer 13c. The three phases have a distinct interface, as shown in fig. 3, and can subsequently be drained off as mainly pure phases one after the other. The aqueous phase 23 passes during the draining off through the solid phase 22 and is drained off in the same way as the first phase through the hydrolysate outlet 16.

The solid phase acts in this way during the draining off of the above resting phases as a filter and holds imparities back. Therefore the aqueous phase leaves the hydrolysate outlet without visible impurities. The drained off aqueous phase optionally passes further through a fat separation tank and is collected. The content of proteins, peptides and amino acids can be extracted, or the aqueous hydrolysate phase can without further processing be used as for example soup or meat extract.

Thereafter, the fat component is led off in the same manner via the hydrolysate outlet 16, however, a part of the fat settles as rest fat on the bones.

This rest fat and other particulate material is let off at the end together with the solid phase through the bone outlet 20, which is opened and closed by the slide valve 18.

The reactor 2 is easy to wash. The reactor, on whose bottom or sides there perhaps will be sediment or coatings, respectively, is filled with water and if necessary detergent. The mixers 13 are started and the washing mixture washes the inner side of the reactor 2 until the dirty washing water is drained off through either the hydrolysate outlet 16 or the bone outlet 18, or through both.

Preferably the shovel blades are formed such that the solid phase with the bone waste is shoved back and forth on the bottom of the trough, when the mixing is reversible, and is pushed towards an outlet, when the same rotation direction is maintained.

The mixers are in the figure shown with half circular formed shovel blades. The shape of the shovel blades is however not limited to that shown in the drawing. Within the scope of the invention, the shovel blades can e.g. be edged, have bends, or be mounted at an angle to the mixer in relation to its axis.

### ILLUSTRATION EXAMPLE

A trough formed insulated reactor, which is equipped with a heating jacket, has a width of 1.4m, a length of 2.8m, and a capacity of 5,300 litres. The reactor is filled with a raw material reaction mixture of 3,000kg pork bones from the slaughterhouse waste (broken up into pieces of approximately 5 cm's in length) and 2,000kg water. It is mixed with a rotation speed of six rotations per minute. The rotation direction of the mixers is changed each minute. The heat jacket contains steam at 2 bar. Heat is applied until the mixture has an average temperature of 55 °C. Thereafter, 3,000g of Novozyme 2,4 Alcalase (protease) is added and the mixing is maintain for 1 hour. The hydrolysis process is stopped by heat denaturizing the Alcalase by raising the reaction mixtures temperature to 95 °C in 25 minutes using the steam jacket.

The mixing is stopped and the reaction mixture rests for 10 minutes. The bone fraction sinks to the bottom of the reactor. First approximately 3,000 L of clear liquid is tapped with a protein content of 6 w% calculated on the basis of the weight of pork bone. Then 12 w% clear liquid fat is drained off, calculated on basis of the mass of pork bone.

The remaining content in the reactor is discharges through the bone outlet by means of the rotating mixers pushing the bones through the open second valve and down into a bone vat.

## Claims

1. A plant (1) for enzymatic hydrolysis of a batch of mainly partly hydrolysable animal or vegetable components or a mixture of these components, which comprises a reactor (2) with a mixer (13) and at least one outlet (16,20), wherein the reactor (2) has an open top (7) and a bottom (10), **characterized in that** the reactor (2) is formed as a round bottom trough (2) with two opposite side walls (11,12) and a first (8) and second (9) opposite end wall, a rotatable axle (15) suspended between the end walls (8,9) at a distance from the bottom (10), and at least one mixer (13) arranged on the axle (15).

2. A batch plant (1) according to claim 1, **characterized in that** the trough (2), axle (15) and the at least one mixer (13) is dimensioned in relation to each other such that,
- the at least one mixer (13) is extending mainly symmetrically around the axis of the axle (15) towards the bottom (10) of the trough (2) with a length x, which is equal to or a little smaller than the distance between the axis of the axle (15) and the deepest point of the trough (2), and
- that the depth h of the trough (2) is so much larger than the total length (2x) of the at least one mixer (13) that the fat phase (24) from an aqueous enzymatic hydrolysis of a batch of hydrolysable animal or vegetable components or a mixture of these components, rests above the rotation top point of the at least one mixer (13) at a distance (y), which is larger than zero.

3. A batch plant (1) according to claim 1 or 2, **characterized in that** a number of mixers (13) are arranged on the axle (15) with a mainly equidistant spacing (b) between each other.

4. A batch plant (1) according to claim 1, 2 or 3, **characterized in that** the mixers (13) are mounted on the axle (15) angularly displaced around the axis of the axle (15) in relation to each other.

5. A batch plant (1) according to any one of claims 1 - 4, **characterized in,**
- **that** the outlet (16) is a hydrolysate outlet (16) for discharing the liquid (23) from the trough (2),
- **that** the hydrolysate outlet (16) is formed in one of the side walls (11,12) close to the bottom (10) of the trough,
- **that** the hydrolysate outlet (16) is arranged with a first valve (17), and
- **that** the distance between the end wall of the reactor and the first valve (17) is less than or equal to 50 cm, preferably less than or equal to 30 cm, and most preferably less than or equal to 15 cm.

6. A batch plant (1) according to claim 5, **characterized in that** the hydrolysate outlet (16) has a minimum diameter of approximately 5 cm.

7. A batch plant (1) according to any one of claims 1 - 6, **characterized in,**
- **that** the second outlet (20) is a bone outlet (20) for discharging the solid phase (22),
- **that** the bone outlet (20) is formed in the bottom (10) of the trough (2) near an end wall, and
- **that** the bone outlet (20) has a second valve (18).

8. A batch plant (1) according to any one of claims 1 - 7, **characterized in that**, the bone outlet (20) is used as the hydrolysate outlet (16).

9. A method to produce a protein containing hydrolysate (23) of an aqueous mixture of at least partly hydrolysable animal or vegetable components or a mixture thereof, wherein the method uses a batch plant (1) according to any one of claims 1 - 8 and comprises the steps of:
- heating the mixture (21) to a hydrolysis temperature,
- adding a protease or a mixture of proteases, in order to hydrolyse the content of hydrolysable material of the mixture (21),
- mixing the reaction mixture using a mixer,
- proteolyzing hydrolysable components in the mixture (21) in a holding time during mixing,
- inactivating the protease or the mixture of the proteases with heat denaturation, **characterized in that**,
- the rotation of the mixers (13) is stopped,
- the reaction mixture (21) rests until phase separation into at least three phases, a solid phase (22), a aqueous phase (23) and a fat phase (24), respectively,
- a first draining off step is carried out to drain off the aqueous phase (23) by passage through the solid phase (22) and out through the hydrolysat outlet (16), and
- a second draining step is carried out to led off the fat phase (24) by passage through the solid phase (22) and out through the bone outlet (20).

10. A method according to claim 9, **characterized in that** the method comprises a third draining off step wherein the mixers (13) rotate the solid phase (22) out through the bone outlet (17)

## Patentansprüche

1. Eine Anlage (1) zur enzymatischen Hydrolyse eines Batch von wenigstens teilweise hydrolysierbaren tierischen oder pflanzlichen Bestandteilen oder einer Mischung aus diesen Bestandteilen, die einen Reaktor (2) mit einem Mischer (13) und wenigstens einem Auslass (16, 20) aufweist, wobei die Oberseite (7) des Reaktors (2) offen ist und der Reaktor (2) einen Boden (10) hat, **dadurch gekennzeichnet, dass** der Reaktor als ein Trog (2) mit einem runden Boden, zwei einander gegenüber liegenden Seitenwänden (11, 12) und einer ersten (8) und einer zweiten (9) einander gegenüber liegenden Stirnwandungen aufgebildet ist, eine drehbare Welle (15) zwischen den Stirnwänden (8, 9) mit einem Abstand von dem Boden (10) aufgehängt ist und wenigstens ein Mischer (13) an der Welle (15) angeordnet ist.

2. Eine Batchanlage (1) nach Anspruch 1, **dadurch gekennzeichnet, dass** der Trog (2) die Welle (15) und der wenigstens eine Mischer (13) in Bezug aufeinander derart dimensioniert sind, dass
- der wenigstens eine Mischer (13) sich im Wesentlichen symmetrisch um die Achse der Welle (15) in Richtung auf den Boden (10) der Trog (2) mit einer Länge x erstreckt, die gleich ist oder ein wenig geringer als der Abstand zwischen der Achse der Welle (15) und dem tiefsten Punkt der Trog (2), und
- dass die Tiefe h des Troges (2) so viel größer ist als die Gesamtlänge (2x) des wenigstens einen Mischers (13) ist, dass die Fettphase (24) aus einer flüssigen enzymatischen Hydrolyse eines Batches von hydrolysierbaren tierischen oder pflanzlichen Komponenten oder einer Mischung aus diesen Komponenten sich oberhalb eines oberen Drehpunktes des wenigstens einen Mischers (13) mit einem Abstand (y) absetzt, der größer als Null ist.

3. Eine Batchanlage (1) nach Anspruch 1 oder 2, **dadurch gekennzeichnet, dass** eine Anzahl von Mischern (13) an der Welle (15) mit einem im Wesentlichen gleichen Abstand (b) zwischen einander angeordnet ist.

4. Eine Batchanlage (1) nach einem der Ansprüche 1, 2 oder 3, **dadurch gekennzeichnet, dass** die Mischer (13) auf der Welle (15) axial um die Achse der Welle (15) in Bezug aufeinander versetzt angeordnet sind.

5. Eine Batchanlage (1) nach einem der Ansprüche 1-4, **dadurch gekennzeichnet, dass**
- der Auslass (16) ein Hydrolysatauslass (16) zum Abführen der Flüssigkeit (23) aus dem Trog (2) ist,
- der Hydrolyseauslass (16) in einem der Seitenwände (11, 12) nahe dem Boden (10) der Trog ausgebildet ist;
- der Hydrolyseauslass (16) mit einem ersten Ventil (17) versehen ist, und
- der Abstand zwischen der Stirnwand des Reaktors und dem ersten Ventil (17) gleich oder kleiner ist als 50cm, vorzugsweise kleiner als oder gleich 30 cm und besonders bevorzugt kleiner oder gleich 15 cm.

6. Eine Batchanlage (1) nach Anspruch 5, **dadurch gekennzeichnet, dass** der Hydrolysatauslass (16) einen minimalen Durchmesser von etwa 5 cm hat.

7. Eine Batchanlage (1) nach einem der Ansprüche 1-6, **dadurch gekennzeichnet, dass**
- der zweite Auslass (20) ein Knochenauslass (20) zum Abführen der Festphase (22) ist,
- der Knochenauslass (20) in dem Boden (10) des Trogs (2) nahe an einer Stirnwand angeordnet ist, und
- der Knochenauslass (20) ein zweites Ventil (18) hat.

8. Eine Batchanlage (1) nach einem der Ansprüche 1-7, **dadurch gekennzeichnet, dass** der Knochenauslass (20) als Hydrolyseauslass (16) verwendet wird.

9. Ein Verfahren zum Vermischen von wenigstens teilweise hydrolysierbaren tierischen oder pflanzlichen Bestandteilen oder einer Mischung daraus, wobei das Verfahren eine Batchanlage (1) nach einem der Ansprüche 1-8 verwendet und mit den folgenden Schritten:
- Erhitzen der Mischung (21) auf eine Hydrolysetemperatur,
- Hinzufügen einer Protease oder einer Mischung von Proteasen, um den Gehalt an hydrolysierbarem Material der Mischung (21) zu hydrolysieren,
- Vermischen der Reaktionsmischung unter Verwendung eines Mischers,
- Proteolysieren der hydrolysierbaren Bestandteile in der Mischung (21) in einer Haltezeit während des Mischens,
- Inaktivieren der Protease oder einer Mischung der Proteasen durch Hitzedenaturisierung, **dadurch gekennzeichnet, dass**
- die Rotation des Mischers (13) gestoppt wird,
- die Reaktionsmischung (21) ruht, bis die Phasentrennung in wenigstens drei Phasen, eine Festphase (22), eine wässrige Phase (23) und eine Fettphase (24) stattgefunden hat,
- eine erster Drainageschritt zum Abziehen der flüssigen Phase (22) durch Passage durch die flüssige Phase (22) und Abführen durch den Hydrolysatauslass (16) ausgeführt wird und
- ein zweiter Drainageschritt zum Abführen der Fettphase (24) durch Hindurchführen der festen Phase (22) und Abführen durch den Knochenauslass (20) ausgeführt wird.

10. Ein Verfahren nach Anspruch 9, **dadurch gekennzeichnet, dass** das Verfahren weiter einen dritten Drainageschritt aufweist, bei dem die feste Phase (22) bei sich drehenden Mischern (13) durch den Knochenauslass (17) abgeführt wird.

## Revendications

1. Installation (1) pour l'hydrolyse enzymatique d'un lot de composants animaux ou végétaux principalement partiellement hydrolysable ou un mélange de ces composants qui comprend un réacteur (2) avec un mélangeur (13) et au moins une sortie (16, 20), où le réacteur (2) possède un sommet ouvert (7) et un fond (10), **caractérisée en ce que** le réacteur (2) est formé comme une cuve à fond rond (2) avec deux parois latérales opposées (11, 12) et une première (8) et une deuxième paroi d'extrémité opposée (9), un axe rotatif (15) suspendu entre les parois d'extrémité (8, 9) à une distance du fond (10), et au moins un mélangeur (13) agencé sur l'axe (15).

2. Installation discontinue (1) selon la revendication 1, **caractérisée en ce que** la cuve (2), l'axe (15) et l'au moins un mélangeur (13) sont dimensionnés les uns par rapport aux autres de sorte que,
l'au moins un mélangeur (13) s'étende principalement de manière symétrique autour de l'axe de l'axe (15) vers le fond (10) de la cuve (2) avec une longueur x, qui est égale ou légèrement inférieure à la distance entre l'axe de l'axe (15) et le point le plus profond de la cuve (2), et que
la profondeur h de la cuve (2) soit beaucoup plus grande que la longueur totale (2x) de l'au moins un mélangeur (13) de sorte que la phase graisseuse (24) provenant d'une hydrolyse enzymatique aqueuse d'un lot de composants animaux ou végétaux hydrolysables ou un mélange de ces composants, repose au-dessus du point supérieur de rotation de l'au moins un mélangeur (13) à une distance (y), qui est supérieure à zéro.

3. Installation discontinue (1) selon la revendication 1 ou 2, **caractérisée en ce qu'**un nombre de mélangeurs (13) sont agencés sur l'axe (15) avec un espacement principalement équidistant (b) entre eux.

4. Installation discontinue (1) selon la revendication 1, 2 ou 3, **caractérisée en ce que** les mélangeurs (13) sont montés sur l'axe (15) déplacés de manière angulaire autour de l'axe de l'axe (15) en relation les uns par rapport aux autres.

5. Installation discontinue (1) selon l'une quelconque des revendications 1 à 4, **caractérisée en ce que**,
la sortie (16) est une sortie d'hydrolysat (16) pour évacuer le liquide (23) de la cuve (2),
la sortie d'hydrolysat (16) est formée dans une des parois latérales (11, 12) à proximité du fond (10) de la cuve,
la sortie d'hydrolysat (16) est agencée avec une première vanne (17), et
la distance entre la paroi d'extrémité du réacteur et la première vanne (17) est inférieure ou égale à 50 cm, de préférence inférieure ou égale à 30 cm, et de manière plus préférée inférieure ou égale à 15 cm.

6. Installation discontinue (1) selon la revendication 5, **caractérisée en ce que** la sortie d'hydrolysat (16) possède un diamètre minimum d'approximativement 5 cm.

7. Installation discontinue (1) selon l'une quelconque des revendications 1 à 6, **caractérisée en ce que**,
la deuxième sortie (20) est une sortie d'os (20) pour évacuer la phase solide (22),
la sortie d'os (20) est formée dans le fond (10) de la cuve (2) à proximité d'une paroi d'extrémité, et
la sortie d'os (20) possède une deuxième vanne (18).

8. Installation discontinue (1) selon l'une quelconque des revendications 1 à 7, **caractérisée en ce que** la sortie d'os (20) est utilisée en tant que la sortie d'hydrolysat (16).

9. Procédé pour produire un hydrolysat contenant des protéines (23) d'un mélange aqueux de composants animaux ou végétaux au moins partiellement hydrolysables ou un mélange de ceux-ci, lequel procédé utilise une installation discontinue (1) selon l'une quelconque des revendications 1 à 8, et comprend les étapes consistant à :
chauffer le mélange (21) à une température d'hydrolyse,
ajouter une protéase ou un mélange de protéases, afin d'hydrolyser le contenu du matériau hydrolysable du mélange (21),
mélanger le mélange réactionnel en utilisant un mélangeur,
protéolyser les composants hydrolysables dans le mélange (21) pendant un temps de séjour durant le mélange,
inactiver la protéase ou le mélange des protéases par dénaturation thermique, **caractérisé en ce que**,
la rotation des mélangeurs (13) est stoppée,
le mélange réactionnel (21) repose jusqu'à la séparation de phase en au moins trois phases, une phase solide (22), une phase aqueuse (23) et une phase graisseuse (24), respectivement,
une première étape de drainage est réalisée pour évacuer la phase aqueuse (23) par passage à travers la phase solide (22) et vidange par la sortie d'hydrolysat (16), et
une deuxième étape de drainage est réalisée pour faire sortir la phase de graisse (24) par passage à travers la phase solide (22) et évacuation par la sortie d'os (20).

10. Procédé selon la revendication 9, **caractérisé en ce que** le procédé comprend une troisième étape d'évacuation où les mélangeurs (13) font tourner la phase solide (22) pour la faire sortir par la sortie d'os (17).
